# EUROPEAN PATENT APPLICATION

(11) **EP 1 080 735 A1**
(43) Date of publication of application: **07.03.2001**
(21) Application number: 99122908.9
(22) Date of filing: 18.11.1999
(51) Int. Cl.: A61L 15/60

(54) **Granular liquid absorbent material**

(30) Priority: 03.09.1999 JP 24981599
(71) Applicant: Komine, Keiji, Funabashi-shi, Chiba (JP); Ishigaki, Ryuzo, Koshigaya-shi, Saitama (JP)
(72) Inventor: Komine, Keiji, Funabashi-shi, Chiba (JP); Ishigaki, Ryuzo, Koshigaya-shi, Saitama (JP)
(74) Representative: Patentanwälte Lippert, Stachow, Schmidt & Partner

(57) **Abstract**

A granular liquid absorbent material prepared by mixing a powdery water absorbing polymer as a main ingredient and a powdery cellulosic binder the function of which as the disintegrate is improved when used in combination with a swelling agent such as a water absorbing polymer, applying a strong pressure to the mixture and shaping them under compression into a granular solid material, which is inserted into a urine discharge tube 8 of a pouch 7 for a urinary tract stoma, the granular liquid absorbent material capable of rapidly gelling watery feces or urine discharged in the pouch and being filled easily from the urine discharge tube to the inside of the pouch.

## Description

### BACKGROUND OF THE INVENTION

### Field of the invention

The present invention concerns a granular liquid absorbent material suitable for immobilization by gelation of urine or feces discharged in a pouch for a stoma attached to a discharging port of a digestive tract stoma (artificial anus) or urinary tract stoma (artificial bladder) or in a portable toilet, for gelation of spittles spitted in a cuspidor or a basin thereby making them less overflowing, or for formation of water stopping barriers to a water inundated or flooded portion of a building or for application of temporary water proof repair to cracked portions.

### Related Art Statement

A pouch for a stoma for the recovery of excrements such as feces or urine discharged in voluntarily from a stoma formed to an abdominal wall generally includes those which are appended to the abdominal wall by an adhesive at the periphery of an attaching port thereof formed with a circular hole facing the discharge aperture of the stoma. However, when a user (stomate) walks, stands-up or sits-down while carrying about the attached stoma, there may be a worry that liquids such as watery feces or urine discharged to the inside of the pouch shake like waving to leak the liquids or bad smells from the attaching port of the pouch to the outside, the liquids are deposit to the stomate's muscosa to cause sore, or the liquids deposit to the adhesive used for appending the stoma to the abdominal wall to deteriorate the adhesion and cause the pouch to droop out of the abdominal wall.

Further, feces or urine accumulated inside of the stoma pouch are discharged from a drain hole or a drain tube such as a urine discharge tube disposed at the attaching port of the pouch or at the bottom of the pouch and are discarded, for example, into a toilet stool. In the course of such disposal, it is a worry in that liquid droplets of watery feces or urine may possibly scatter around the periphery thereby polluting the stomate's body or wearing or contaminating the surface of the toilet stool and the floor surface of the toilet.

In view of the above, it has been proposed an invention in which a powder of a water absorbing resin for gelling liquids excrements such as watery feces are filled from the attaching port to the inside of the pouch for a digestive tract stoma, or a powder of the absorbing resin sealed in a bag made of a water soluble paper into a stick-like shape are incorporated (Japanese Patent Laid-Open Hei-5-176950). However, this invention has no concerns with rapid gelation of liquid excrements discharged to the inside of the bag of the pouch.

That is, since the powder of the water absorbing resin charged to the inside and accumulated at the bottom of the pouch for the stoma is swollen and gelled only at the portion of the surface layer which is at first brought into contact with the liquid excrements discharged out of the discharge port of the stoma and forms a barrier of jelly at the portion of the surface layer that inhibits penetration of liquid excrements, it is less disintegratable entirely and takes much time to be dissolved into the liquids of the excrements.

Particularly, since the powdery liquid absorbing resin sealed in a bag made of water soluble paper into a stick-like shape is further less integratable, it can not rapidly gel the liquid excrements.

Further, the powder of the water absorbing resin can not be filled from the attaching port of the pouch in a state of attaching the pouch for the stoma to an abdominal wall, and, even before attaching the pouch, it is also extremely difficult for a stomate to fill the powder of the water absorbing resin from the attaching port of stomate without spilling powder. Further, the powder of the water absorbing resin to be filled from the attaching port of the pouch may deposit to the periphery of the attaching port or scatter inside of the pouch and transfer and deposit to the skin of the abdominal walls or the muscosa of the stomate thereby possibly causing inflammation such as eruption or sore.

Further, in a pouch for a urinary tract stoma of a type in which a check valve is formed to a central part of the pouch such that urine accumulated in the inside thereof does not flow backwardly to the attaching port, the powder of the absorbing resin can not be filled from the attaching port because of hindrance given by the check valve.

In view of the above, the present applicant has developed a sheet-like liquid absorbent 21 as shown in Fig. 1 - Fig. 6 (U.S. Patent No. 5,786,056).

In the liquid absorbent material 21, surface layers 22 and 22 are formed of soft water soluble paper or water dispersible paper which is dispersed and dissolved in a short time into liquids, an intermediate layer 23 formed as a laminate sheet S of a three layered structure comprising a web prepared by mixing a highly water absorbing fibrous cellulosic resin and a powdery highly water absorbing synthetic polymeric resin 25 and bonding them with a thermoadhesive and water soluble powdery adhesive 26 in which a plurality of linear parallel recesses 27 are arranged each at a predetermined pitch in the lateral direction of the laminate sheet S, such that the laminate sheet S can be used by being torn to such an appropriate size that can be inserted into the inside of the pouch for the stoma

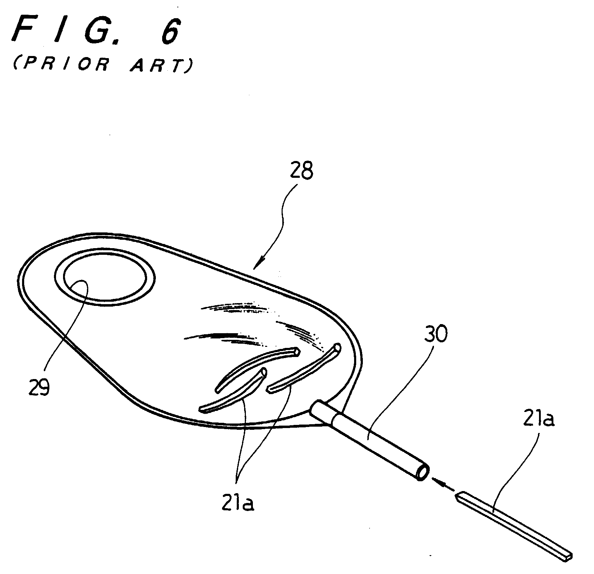

That is, the liquid absorbent material 21 can be inserted from the attaching port 29 to the inside of a pouch 28 for a urinary tract stoma as shown in Fig. 6 by being torn into an appropriate width and crumpled. A stick-shaped liquid absorbent material 21a formed by tearing the stick into a minimum width unit can be inserted also front an elongate narrow urine discharge tube 30 disposed at the bottom of the pouch 28 for the urinary tract stoma, and an appropriate amount of the liquid absorbent material can be filled according to the inner capacity of the pouch 28 by controlling the number of pieces to be of inserted.

Then, since the intermediate layer 23 of the laminate sheet S is formed of the web in which water absorbing fibrous cellulosic resin 24 is overlapped while being entangled with each other, liquid excrements are rapidly penetrate under the capillary action of the web, so that the powdery water absorbing synthetic polymeric resin 25 can uniformly absorb water and swell entirely to rapidly gel the liquid excrements.

Further, since the powdery water absorbing synthetic polymeric resin 25 is disposed to the intermediate layer 23 put between the surface layers 22 and 23 formed of water soluble paper or paper dispersible paper and since it is set together with the water absorbing fibrous cellulosic resin 24 with a water soluble powdery adhesive 26, there is no worry that the powder of the water absorbing synthetic polymeric resin 25 should transfer and deposit to the skin of the abdominal wall or the muscosa of a stomate to cause inflammation such as eruption or sore.

Accordingly, the sheet-like liquid absorbent material 21 has been popularized generally and rapidly at the time of starting the sale of the products, which have now been used favorably by many stomates.

However, the liquid absorbent material 21 involves a problem that the production cost is expensive and the productivity is not so high, because fabrication of forming the liquid absorbent material 21 into the laminate sheet S of the three layered structure comprising the surface layers 22, 22 and the intermediate layer 23, or forming a plurality of recessed 27 to the laminate sheet S are troublesome.

Further, since the liquid absorbent material 21 formed of the laminate sheet S is soft and lacks in rigidity, it is difficult to successively enter stick-shaped liquid absorbent material 21a formed by finely tearing the material into an elongate narrow liquid discharge tube 30 of the pouch 28 for the urinary tract stoma continuously and rapidly fill to the inside of the pouch 28, and it takes a considerable period of time for filling a necessary amount of the liquid absorbent material 21a. Furthermore, when the liquid absorbent material 21a is filled to the inside of the pouch 28, the water soluble paper or water dispersible paper of the surface layers 22, 22 may not sometimes be dissolved completely in a gel but keep the shape of a strip after gelling of urine and clog the urine discharge pipe 30 of the pouch 28 making it difficult to release gelled urine through the urine discharge tube.

Furthermore, in the liquid absorbent material 21 since the powder of the synthetic polymeric water absorbing resin 25 of high water absorption factor is disposed being dispersed in a bulky web forming the intermediate layer 23 of the laminate sheet S, the bulk density of the synthetic polymeric water absorbing resin 25 is low. Accordingly, it also gives rise to a problem that a necessary amount for the pouch can not be obtained unless a great amount of the resin is filled to the inside of the pouch for the stoma.

### OBJECT OF THE INVENTION

In view of the above, it is an object of the present invention to provide a liquid absorbent material capable of rapidly gelling a liquid, satisfying the required amount in a pouch for a stoma by using a small amount of the material, capable of being easily and rapidly filled from a urine discharge tube of the pouch for a urinary tract stoma to the inside of the pouch, and capable of discharging gelled urine from a urine discharge tube with no worry of clogging the urine discharge tube, and enable mass production of the liquid absorbent material at a reduced cost.

### SUMMARY OF THE INVENTION

The foregoing object of the present invention can be attained in a granular absorbent material comprising a powder of a powdery water absorbing polymer as a main ingredient and a powdery cellulosic binder mixed therewith which is pressed strongly in a dry state as it is to shape the powder under compression into a granular solid material.

The liquid absorbent material formed of the granular solid material when shaped into such a size as capable of being inserted into a liquid discharge tube of a pouch for a urinary tract stoma, can be forced successively into the urine discharge tube and can be filled easily and rapidly in the inside of the pouch. Further, since this is formed only of the powder of the water absorbing polymer and the cellulosic binder, without using water soluble paper or water dispersible paper, there is no worry of causing clogging to the liquid discharge tube when urine gelled in the inside of the pouch for the urinary tract stoma is discharged from the urine discharge tube.

Further, since the granular liquid absorbent material is formed by compressing the powdery water absorbing polymer as the main ingredient, the water absorbing polymer has a high bulk density and high water absorbing capacity even if the size if small, so that the amount required for the pouch can be satisfied by merely filling the same in a small amount to the pouch for the stoma. Further, the amount of the granular liquid absorbent material to be used can be controlled simply and precisely by the number of the material.

Further, the powdery cellulosic binder mixed in the powdery water absorbing polymer functions as a binder for the powder when the powders are shaped under compression into the granular solid material. In addition, when the binder is in contact with a liquid, it also functions as a disintegrator for disintegrating the granular solids, the powder of the water absorbing polymer as the main ingredient of the granular liquid absorbent material according to the present invention can be dissolved uniformly in the liquid to rapidly gel the liquid.

Moreover, since the function of the cellulosic binder as the disintegrator can be improved further by the combined use with a swelling agent such as the water absorbing polymer, it is possible to rapidly disintegrate the granular liquid absorbent material entirely in contact with the liquid and dissolve the water absorbing polymer rapidly into the liquid.

Furthermore, since the granular liquid absorbent material according to the present invention can be manufactured by a simple fabrication process of applying a strong pressure to the powder formed by mixing the powdery water absorbing polymer and the powdery cellulosic binder in a dried state as it is and shaping the powder mixture into the granular solid material under compression, the productivity is high and the material cost and fabrication cost are inexpensive. Accordingly, the material an be provided at a reduced cost to stomates.

### DESCRIPTION OF THE ACCOMPANYING DRAWINGS

Fig. 1 is a view showing an example of a granular liquid absorbent material according to the present invention;
Fig. 2 is a view schematically showing an example of a manufacturing method of the granular liquid absorbent material;
Fig. 3 is a view showing an example of a method of using the granular liquid absorbent material;

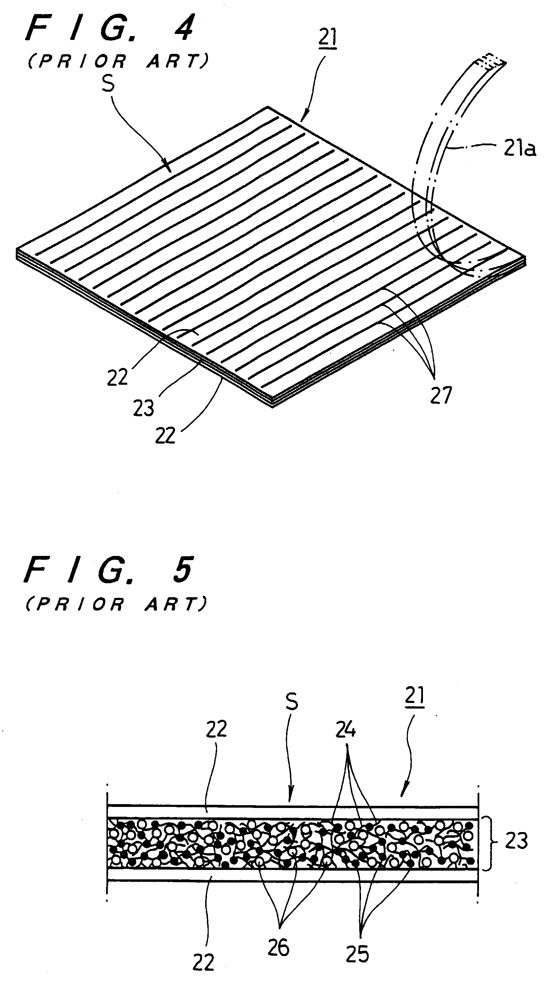

Fig. 4 is a view illustrating an entire structure of an existent liquid absorbent material;
Fig. 5 is a view showing a cross sectional structure of the existent liquid absorbent material; and
Fig. 6 is a view showing a method of using the existent liquid absorbent material

### DESCRIPTION OF PREFERRED EMBODIMENT

A preferred embodiment of the present invention is to be explained more in detail specifically referring to the drawings.

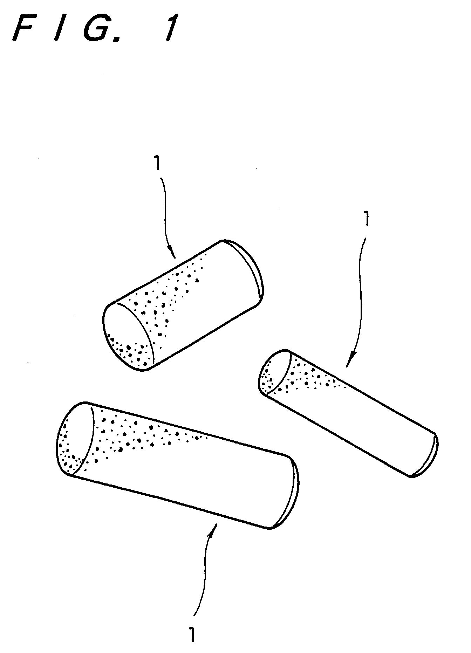

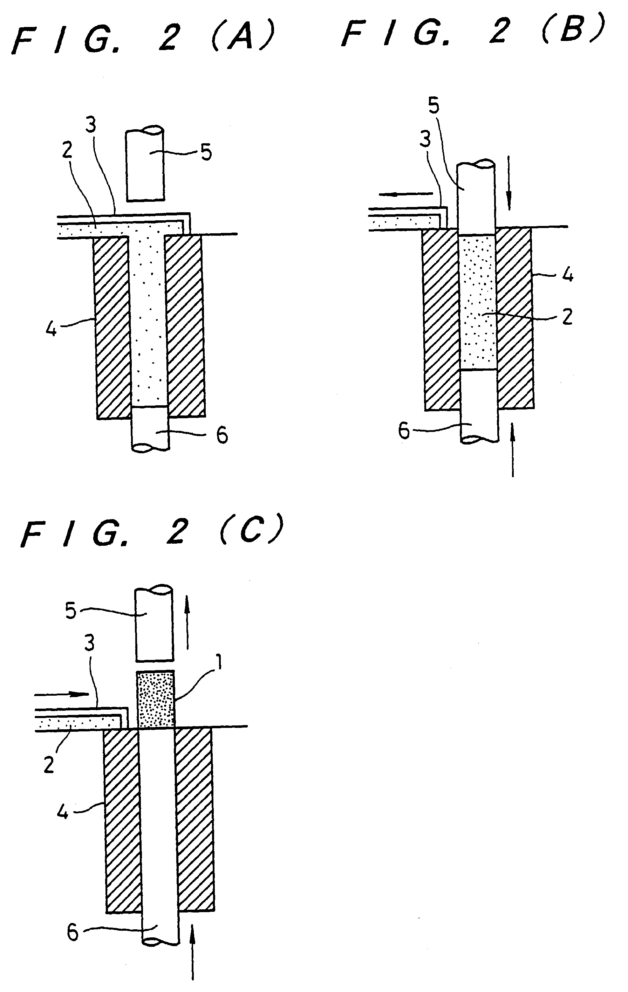

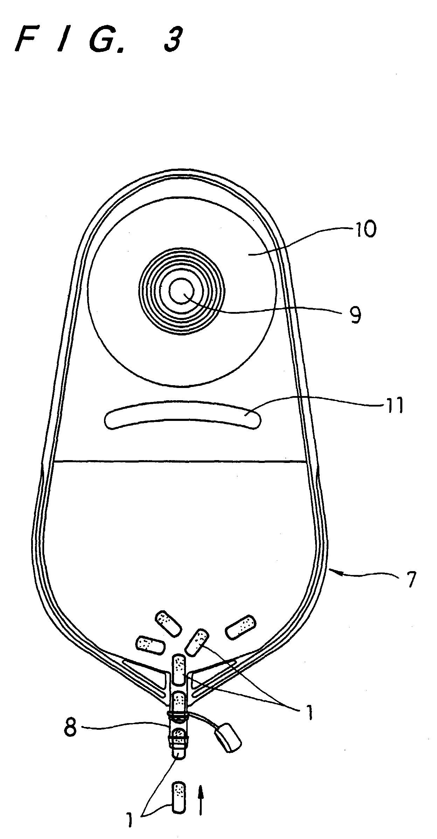

Fig. 1 is a view showing an example of a granular absorbent material according to the present invention, Fig. 2 is a view schematically showing an example of a manufacturing method of the granular liquid absorbent material, and Fig. 3 is a view showing an example of a method of using the granular liquid absorbent material.

For example, as shown in Fig. 2, a granular liquid absorbent material 1 is formed by filling a powder 2 comprising a mixture of a powdery cellulosic binder and a powdery liquid absorbing polymer as the main ingredient in a dried state as it is into a cylindrical mortar 4 by a feeder 3 (Fig. 1A), a strong pressure is applied in the vertical direction by both of an upper pounder 5 and a lower pounder 6 to the powder 2 filled in the mortar 4 (Fig. 1B) to shape the powder 2 into a granular solid material under compression (Fig. 1C).

Further, the granular liquid absorbent material is formed into a substantially columnar shape of 4 to 8 mm diameter and 10 to 60 mm length such that the shaped material can be forced one by one to an elongate narrow urine discharge tube 8 of 5 to 9 mm inner diameter disposed at the bottom of a pouch 7 for a urinary tract stoma and can be inserted easily and rapidly to the inside of the pouch 7. The material is molded under compression to a hardness of 3 to 6 kg as measured by a Monsanto hardness gage such that the material is not crushed or chipped easily by such an external force as exerted when the material is pinched by fingers.

That is, the pouch 7 for a urinary tract stoma is a commercial product of a type in which a doughnuts-shaped flange 10 is disposed at the periphery of an attaching port 9 facing the discharge port of the stoma. An adhesive is coated on the surface of the flange and releasing paper is appended thereon, and the flange is appended to an abdominal wall with the adhesive coated on the surface thereof. In the attached state, the liquid absorbent material can not be inserted from the attaching port 9. Further, since a strip-shaped fusion portion 11 is disposed for thermally fusing both of the surface and the rearface of the pouch 7 made of a thermoplastic sheet for forming a check valve at the central part of the pouch 7 that prevents back flow of urine, it is difficult to fill the liquid absorbent material from the attaching port to the bottom of the pouch 7.

In view of the above, the granular liquid absorbent material 1 is shaped to such a form and a size that it can be picked-up with fingers and easily inserted into the tube of the urine discharge tube 8, and the material is shaped under compression to such a hardness as not giving any worry of being crushed or chipped upon entry.

For the water absorbing polymer as the main ingredient of the granular liquid absorbent material 1, it is usable a polyacrylic acid type water absorbing polymer having a water absorption performance of about 150 to 1000 times its own weight relative to distilled water, and about 30 to 60 times by weight relative to physiological saline, or an amino acid type water absorbing polymer having about 500 to 1000 times its own weight relative to distilled water and 50 times or more its own weight relative to the physiological saline.

Further, for the cellulosic binder to be mixed with the water absorbing polymer, there can be used, for example, methyl cellulose (MC), carboxymethyl cellulose (CMC), hydroxypropyl cellulose (HPC) or hydroxypropylmethyl cellulose (HPMC).

According to the experiment, a commercially available cellulosic binder most suitable to the granular liquid absorbent material according to the present invention is powdery cellulose manufactured by Rettenmaier & Söhne GMBH in Germany (trade name of products "VITACEL" comprising highly pure cellulose (polymerization degree: about 1000) with an average grain size of 30 to 300 µm pulverized mechanically by a dry process. Since this material has a number of hydroxy groups promoting disintegration under the presence of water content and since the disintegration property is increased extremely in combined with a swelling agent such as a water absorbing polymer, the granular liquid absorbent material can be disintegrated instantaneously without using other disintegrating agent such as sodium carboxymethyl starch.

On the contrary, crystalline cellulose (MCC) mechanically pulverized in a fabrication process using an acid and a wet process by which molecules are destroyed to lower the polymerization degree to about 200 to 300 has a sufficient function as the binder but lacks in the function as the disintegrator, so that this cellulosic binder is not suitable to the granular liquid absorbent material according to the present invention. However, since the granular liquid absorbent material can be provided with disintegratability when used in combination with other disintegrator such as starches or sodium carboxymethyl starch, use of the crystalline cellulose as the cellulosic binder is not excluded in the present invention.

Then, it is preferred to select the content of the water absorbing polymer as the main ingredient of the granular liquid absorbent material within a range from 50 to 90% by weight and the content of the cellulosic binder to be mixed therewith within a range from 10 to 50% by weight. If the content of the water absorbing polymer is excessive or insufficient with respect to the range described above, failure tends to be caused in shaping the granular liquid absorbent material. Further, if the content of the water absorbing polymer is insufficient, the water absorbing capacity of the granular liquid absorbent material is decreased to lower the effect of gelling the liquid. On the other hand, if the content is excessive, only the surface portion of the granular liquid absorbent material in contact with the liquid is gelled to form a barrier of jelly, which inhibits the penetration of the liquid at the surface portion, so that the disintegrating speed of the granular liquid absorbent material is retarded and it takes a long time for the powder of the water absorbing polymer to be dissolved uniformly in the liquid.

Further, the average grain size of the water absorbing polymer is optimally about from 150 to 300 µm and, if the average grain size is too large or too small, the fabrication for shaping the granular liquid absorbent material becomes difficult tending to cause failure in shaping. Further, if the average grain size is too small, the granular liquid absorbent material is gelled only at the surface portion making it difficult to be disintegrated as a whole. On the other hand, if the average grain size is too large, hardness of the granular liquid absorbent material is lowered making the material fragile as a whole and it is easily crushed or chipped even by merely pinching and slightly pressing the material by fingers, so that it is inconvenient to use and difficult to handle with.

When a powdery deodorant comprising green tea extracts prepared by extracting leaves of Japanese tea into hot water, purified with ethanol and then drying into a powdery form (manufactured by Maruzen Seiyaku Co.) is added in an amount from 1 to 5% by weight, to the powder 2 prepared by mixing the water absorbing polymer and the cellulosic binder, odors of feces or ammonia in excrements gelled by the granular absorbent material 1 comprising the powder 2 is reduced remarkably.

The green tea extracts, when added only by 1% to a liquid containing 75 ppm of ammonia, show an excellent deodoring effect of lowering the ammonia residue in the liquid to about 3%, as well as have a sterilizing effect due to catechin. Further, it is not harmful to human bodies and if should deposit to the stoma, it does not cause eruption or sore to the ostomate's muscosa, so that they are optimal as the deodorant used for the pouch for the stoma.

Further, when a biodegradable amino acid type water absorbing polymer which is decomposed to about 100% for 60 days in the compost is used as the main ingredient for the granular liquid absorbent material, since the powdery deodorant comprising the cellulosic binder or the green tea extracts to be mixed therewith are also biodegradable organic materials, if excrements such as feces or urine gelled by the granular liquid absorbent material 1 are discarded to sewage, there is no worry of damaging the natural environment at all.

Further, excrements such as feces or urine gelled by the granular liquid absorbent material 1 contain much water and are difficult to be put to the incinerating treatment. However, if the ingredient of the granular liquid absorbent material 1 is an organic material decomposed easily by microorganisms such as amino acid type water absorbing polymer, cellulosic binder or extracts of green tea, the excrements can be put to a compost-forming treatment.

The amino acid type water absorbing polymer includes those water absorbing polymers comprising all kinds of amino acids including aspartic acid as a base skeleton and, for example, a crosslinked polyaspaltic acid formed by using aspartic acid, crosslinking a portion of a polysuccinic acid imide obtained by polymerizing the same with a polyvalent amine such as lysine and putting the remaining imide ring into alkali hydrolysis shows a high water absorption performance substantially equal with that of polyacrylic acid type water absorbing polymer relative to distilled water or physiological saline and, in addition, shows less lowering with lapse of time of the water absorbing amount relative to urine, compared with polyacrylic type water absorbing polymer, so that it is extremely suitable as the main ingredient of the granular liquid absorbent material used as the pouch 7 for the urinary tract stoma.

Further, the granular liquid absorbent material 1, when used in a cuspidor or a basin, it can gel spittles spitted out therein, making them difficult to overflow, can mask odors of spittles and facilitate disposal of spittles. Particularly, the granular liquid absorbent material 1 to which the powdery deodorant comprising the green tea extracts is added can effectively suppress unpleasant smells caused upon petrifaction of the spittles by the sterilizing effect and a deodorizing effect of the green tea extracts.

When a water soluble powdery colorant exhibiting green, blue or purple color giving feeding of clearness in a state of an aqueous solution is added to the powder 2 to form the granular liquid absorbent material 1, spittles with yellow green color spitted out in a cuspidor or a basin are pigmented to different colors to mitigate unpleasant feeling in appearance.

Furthermore, when a portion of a building inundated or flooded with water is covered by the granular liquid absorbent material 1, the granular liquid absorbent 1 is swollen and gelled to form a water stopping barrier. Further, the granular liquid absorbent material 1 can be filled into a cracked portion of concrete structures to apply a temporary water proof repair.

The granular liquid absorbent material is shaped into a substantially columnar shape that can be picked-up by fingers and inserted easily to the urine discharge tube 8 of the pouch 7 for a urinary tract stoma, the granular liquid absorbent material according to the present invention is not restricted only to such a shape but can be shaped into any other shape such as a spherical shape depending on the application use.

### Examples

Then, a best example of the present invention is to be explained.

A powder prepared so as to contain 69% by weight of a water absorbing polyacrylic polymer with an average grain size of about 150 to 250 µm having a water absorbing performance of about 450 times relative to distilled water and 50 times or more relative to physiological saline (trade name of products "ARASORB S 330", manufactured by Arakawa Chemical Industry Co.), and

30% of by weight of highly pure cellulose with an average grain size of about 50 µm (trade name of products "VITACEL P-290" manufactured by Rettenmaier & Söhne GMBH) and

1% by weight of a powdery deodorant comprising extracts of green tea (trade name of products "Green Tea Extract MF", manufactured by Maruzen Seiyaku Co.) is applied with a strong pressure in a dried state as it is and the powdery body is shaped under compression into a substantially columnar solid material of about 6 mm diameter, about 11 mm length and about 0.35 g weight having a hardness of 4 to 5 kg (as measured by Monsanto hardness gage), to obtain a granular liquid absorbent material according to the present invention.

When a granular liquid absorbent material (one piece) is dropped into tap water charged by about 15 cc in a cup, it is disintegrated instantaneously and entirely dissolved uniformly into water within 15 to 18 sec. Simultaneously, the material absorbs water and swells to proceed gelling, by which water in the cup is solidified into a jelly-like block within 28 to 32 sec.

As has been described above, the granular liquid absorbent material according to the present invention, if it is formed into such a size that can be inserted into an elongate narrow urine discharge tube of a pouch for a urinary tract stoma, can be filled easily from the urine discharge tube to the inside of the pouch. Further, since it has a remarkably great water absorbing capacity, it can fill the inside of the pouch with an extremely small amount even if it is of such a small size as can be inserted into the urine discharge tube. Further, when it is in contact with a liquid, it can be disintegrated rapidly, dissolved uniformly in the liquid and rapidly gel the liquid. Further, since it is shaped only with the powdery body without using water soluble paper or water dispersible paper, there is no worry of causing clogging in the urine discharge tube when the urine gelled in the inside of the pouch for the urinary tract stoma is released from the urine discharge tube.

Further, since the liquid absorbent material is formed by shaping a powdery mixture of the water absorbing polymer and the cellulosic binder into a granular solid material, the powder gives no worry at all of transferring and depositing to the skins of the abdominal wall or the muscosa of the stomate to cause inflammation such as eruption or sore.

Further, since the production is easy and the productivity is excellent, it is possible to greatly mitigate the economical burden on stomates by reducing the cost.

## Claims

1. A granular liquid absorbent material comprising a powder of a powdery water absorbing polymer as a main ingredient and a powdery cellulosic binder mixed therewith, which is pressed strongly in a dry state as it is to shape the powder under compression into a granular solid material.

2. A granular liquid absorbent material as defined in claim 1, wherein the water absorbing polymer is a water absorbing polyacrylic acid type polymer.

3. A granular liquid absorbent material as defined in claim 1, wherein the water absorbing polymer is a water absorbing amino acid type polymer.

4. A granular liquid absorbent material as defined in any one of claims 1 to 3, wherein the average grain size of the water absorbing polymer is from 150 to 300 µm.

5. A granular liquid absorbent material as defined in any one of claims 1 to 4, wherein the cellulosic binder is selected from the group consisting of methyl cellulose, carboxymethyl cellulose, hydroxypropyl cellulose or hydroxypropylmethyl cellulose.

6. A granular liquid absorbent material as defined in any one of claims 1 to 5, wherein the content of the water absorbing polymer is from 50 to 90% by weight and the content of the cellulosic binder is from 10 to 50% by weight.

7. A granular liquid absorbent material as defined in any one of claims 1 to 6, wherein a powdery deodorant comprising extracts of green tea prepared by extracting leaves of Japanese tea with hot water and purifying the same with alcohol and then drying them into a powdery form is added by 1 to 5% by weight to the powder.

8. A granular liquid absorbent material as defined in any one of claims 1 to 7, wherein a water soluble powdery colorant is added to the powder.

9. A granular liquid absorbent material as defined in any one of claims 1 to 8, wherein the solid material is shaped substantially into a columnar shape of 4 to 8 mm in diameter and 10 to 60 mm in length.

10. A granular liquid absorbent material as defined in any one of claims 1 to 9, wherein the solid material is shaped under compression to a hardness of 3 to 6 kg (measured by a Monsanto hardness gage).
